# EUROPÄISCHE PATENTANMELDUNG

(11) **EP 1 184 045 A2**
(43) Veröffentlichungstag der Anmeldung: **06.03.2002**
(21) Anmeldenummer: 01250276.1
(22) Anmeldetag: 24.07.2001
(51) Int. Cl.: A61M 1/08

(54) **Schröpfvorrichtung zur punktuelln Behandlung von Hautpartien**

(30) Priorität: 24.07.2000 DE 10037430
(71) Anmelder: Grafischer, Dirk, 14197 Berlin (DE)
(72) Erfinder: Grafischer, Dirk, 14197 Berlin (DE)
(74) Vertreter: Scholz, Hartmut, Dipl.-Ing. Patentanwalt

(57) **Zusammenfassung**

Schröpfvorrichtung (10), bestehend aus einem einseitig offenen, glockenförmigen Schröpfglas (11) zum Aufsetzen auf eine punktuell zu behandelnde Hautpartie. Mit dem Schröpfglas (11) ist ein Evakuator (12) zur Erzeugung eines Unterdrucks im Innenraum (20) des Schröpfglases (11) verbunden. Der Evakuator ist über eine Schröpfglasöffnung (14) mit dem Schröpfglasinnenraum (20) verbunden. In dem Schröpfglasinnenraum (20) ist ein Manipulator (25) angeordnet. Der Manipulator (25) ist als ein in dem Innenraum (20) oberhalb des Schröpfglasrandes (13) querverlaufender Steg ausgebildet.

## Beschreibung

Die Erfindung betrifft eine Schröpfvorrichtung, bestehend aus einem einseitig offenen, glockenförmigen Schröpfglas zum Aufsetzen auf eine punktuell zu behandelnde Hautpartie, einem Evakuator zur Erzeugung eines Unterdrucks im Innenraum des Schröpfglases und einer Schröpfglasöffnung zwischen dem Evakuator und dem Schröpfglasinnenraum.

Derartige Schröpfvorrichtungen, wie sie beispielsweise aus der CH-PS 248 966 bekannt sind, dienen dazu, punktuelle Behandlungen der Haut, insbesondere der menschlichen Haut, vorzunehmen. Mittels eines Evakuators, wie er beispielsweise aus der DE-PS 336 108 bekannt ist, können bestimmte Hautpartien mittels eines in dem Innenraum des Schröpfglases hervorgerufenen Vakuums angesogen werden. Dadurch wird die Haut partiell verdünnt und zu einer besseren Durchblutung angeregt.

Vielfach sollen derartige Therapien durch auf die zu behandelnde Hautpartie aufgetragene Therapeutika unterstützt werden. Problematisch dabei ist es jedoch, daß diese Therapeutika lediglich auf die Oberfläche der Haut, nicht aber darunter geraten.

Aufgabe der vorliegenden Erfindung ist es, eine Schröpfvorrichtung der eingangs beschriebenen Art zu schaffen, mit der Therapeutika durch und unter die Haut appliziert werden können.

Gelöst wird diese Aufgabe dadurch, daß in dem Schröpfglasinnenraum ein Manipulator angeordnet ist. Durch diese Maßnahme wird eine Schröpfvorrichtung geschaffen, bei der mit einem in dem Schröpfglasinnenraum befindlicher Manipulator über die behandelte Hautpartie gestrichen werden kann. Durch dieses Aufstreichen kann ein zuvor auf die Haut aufgetragenes Therapeutikum in das durch den Schröpfvorgang verdünnte Hautgewebe einmassiert werden und gelangt so auch unter die Haut. Der Behandlungserfolg eines Therapeutikums kann dadurch wesentlich erhöht werden.

Weitere vorteilhafte Maßnahmen sind in den Unteransprüchen beschrieben. Die Erfindung ist in der beiliegenden Zeichnung dargestellt und wird nachfolgend näher beschrieben; es zeigt:
- **Figur 1**: den Schnitt durch eine Schröpfvorrichtung in schematischer Darstellung, mit auf eine zu behandelnde Hautpartie aufgesetztem Schröpfglas und auf die angesaugte Hautpartie wirkendem Manipulator;
- **Figur 2**: den Schnitt durch eine Schröpfvorrichtung in schematischer Darstellung, mit auf eine zu behandelnde Hautpartie aufgesetztem Schröpfglas, mit eingeformtem, im Bereich der angesaugten Hautpartie querverlaufendem Manipulator;
- **Figur 3**: die Detaildarstellung eines Manipulators nach der Figur 2, in Draufsicht.

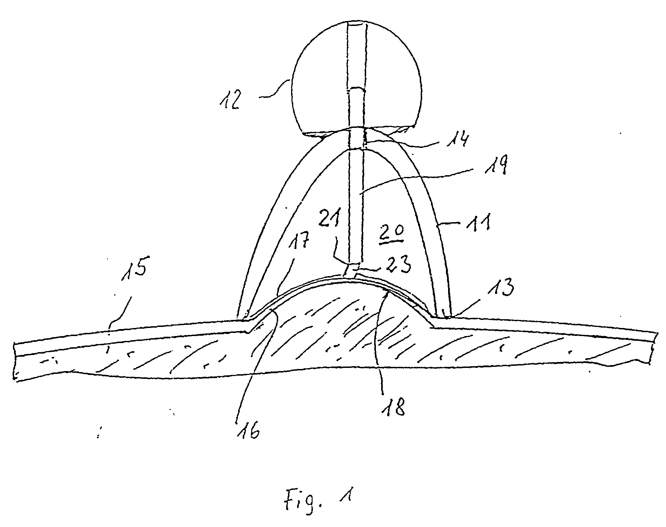

Die in der. Figur 1 schematisch dargestellte Schröpfvorrichtung 10 besteht im wesentlichen aus einem glockenförmigen Schröpfglas 11, welches mit seinem unteren, größeren Schröpfglasrand 13 auf eine zu behandelnde Hautpartie 15 aufgesetzt ist.

Der Innenraum 20 des Schröpfglases 11 kann mittels eines Evakuators 12, beispielsweise einem Gummiballon, durch eine kleinere dem Schröpfglasrand 13 gegenüberliegende obere Schröpfglasöffnung 14 mit einem Unterdruck gegenüber der umgebenden Atmosphäre versehen werden. Durch diesen Unterdruck in dem Innenraum 20 wird die zu behandelnde Hautpartie 16 angesaugt und wölbt sich sphärisch in das Schröpfglas 11 hinein.

Durch die obere Schröpfglasöffnung 14 verläuft ein Manipulator 19, der bis auf die zu behandelnde, angesaugte Hautpartie 16 reicht. Der Manipulator 19 ist flexibel und kann an seiner Unterseite 21 eine hautsympathische Beschichtung 23 aufweisen.

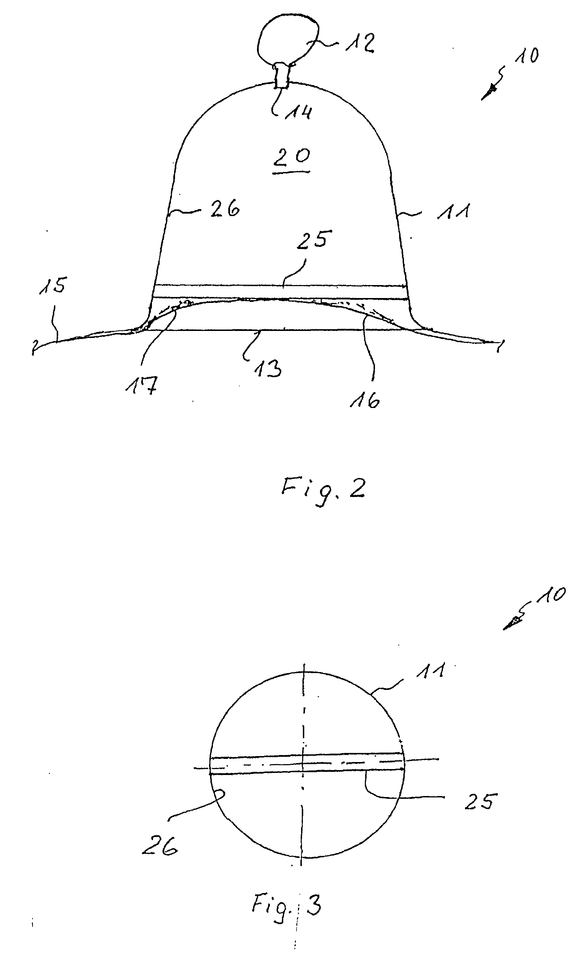

Bei einer anderen, in der Figur 2 dargestellten Ausführung ist der Manipulator 25 als ein zwischen den einander gegenüberliegenden Innenoberflächen 26 des Innenraums 20 verlaufender Steg ausgebildet.

Wie die Figur 3 zeigt, verläuft der Manipulator 25 quer durch den Innenraum 20 des Schröpfglases 11 und ist einstückig mit der Innenoberfläche 26 verbunden. Es ist beispielsweise als starrer runder Glasstab ausgebildet, der aus dem gleichen gläsernen Material wie das Schröpfglas 11 besteht.

Um den Behandlungserfolg zu erhöhen, kann auf die zu behandelnde Hautpartie 15 ein Therapeutikum 17, beispielsweise eine collagenhaltige Salbe, aufgetragen werden. Dieses Therapeutikum 17 kann mit Hilfe des Manipulators 25 auf einer angesaugte Hautpartie 16 verstrichen werden.

Neben einer Anregung der Durchblutung der angesaugten Hautpartie 16 kann durch dieses Verstreichen auch ein Therapeutikum 17 auf die Hautunterseite 18 gelangen. Der Therapieerfolg wird dadurch wesentlich verbessert.

### Bezugszeichen

- 10: Schröpfvorrichtung
- 11: Schröpfglas
- 12: Evakuator
- 13: Schröpfglasrand
- 14: obere Schröpfglasöffnung
- 15: Hautpartie
- 16: angesaugte Hautpartie
- 17: Therapeutikum
- 18: Hautunterseite
- 19: Manipulator
- 20: Innenraum
- 21: Unterseite
- 23: hautsympathische Beschichtung
- 25: Manipulator/Steg
- 26: Innenoberfläche

## Patentansprüche

1. Schröpfvorrichtung, bestehend aus einem einseitig offenen, glockenförmigen Schröpfglas zum Aufsetzen auf eine punktuell zu behandelnde Hautpartie, einem Evakuator zur Erzeugung eines Unterdrucks im Innenraum des Schröpfglases und einer Schröpfglasöffnung zwischen dem Evakuator und dem Schröpfglasinnenraum ein Manipulator angeordnet ist, **dadurch gekennzeichnet, daß** der Manipulator (25) von dem Schröpfglasrand (13) nach innen versetzt querverlaufend in dem Innenraum (20) angeordnet ist.

2. Schröpfvorrichtung nach Anspruch 1, **dadurch gekennzeichnet, daß** der Manipulator (25) an der Innenoberfläche (26) des Innenraums (20) des Schröpfglases (11) angeordnet ist.

3. Schröpfvorrichtung nach den Ansprüchen 1 und 2, **dadurch gekennzeichnet, daß** der Manipulator (25) einstückig mit der Innenoberfläche (26) des Innenraums (20) des Schröpfglases (11) verbunden ist.

4. Schröpfvorrichtung nach den Ansprüchen 1 bis 3, **dadurch gekennzeichnet, daß** der Manipulator (25) als abgerundeter, querverlaufender Steg ausgebildet ist.

5. Schröpfvorrichtung nach den Ansprüchen 1 bis 4, **dadurch gekennzeichnet, daß** der Manipulator (25) starr ist.

6. Schröpfvorrichtung nach den Ansprüchen 1 bis 5, **dadurch gekennzeichnet, daß** der Manipulator (25) eine hautsympathische Beschichtung (23) aufweist.
